# EUROPEAN PATENT SPECIFICATION

(11) **EP 1 951 150 B1**
(45) Date of publication and mention of the grant of the patent: **18.08.2010**
(21) Application number: 05799550.8
(22) Date of filing: 29.10.2005
(51) Int. Cl.: A61F 2/04, A61F 2/84, A61F 2/88

(54) **STENT WITH ANCHORING PORTION**
STENT MIT VERANKERUNGSTEIL
STENT AVEC PARTIE D'ANCRAGE

(43) Date of publication of application: 06.08.2008
(73) Proprietor: PNN Medical SA, 1110 Morges 2 (CH)
(72) Inventor: WALLSTEN, Hans, CH-1135 Denens (CH)
(74) Representative: Vink, Charlotta
(86) International application number: PCT/EP2005/011617
(87) International publication number: WO 2007/048437

(56) References cited:
- EP-A- 0 626 153
- EP-A- 0 723 765
- EP-A- 1 413 262
- WO-A-00/18331
- WO-A-00/66032
- FR-A- 2 661 603
- US-A- 5 514 178
- US-A- 5 540 701
- US-A- 5 667 522
- US-A1- 2001 041 925
- US-B1- 6 416 545

## Description

### Field of the invention

The present invention relates to stents and particularly to the treatment of diseases in the urethra of men.

### Background of the invention

Almost all available stents are so called permanent stents e.g. they are designed to be inserted in occluded vessels and organs to permanently support the vessel and keep it open.

The majority of the permanent stents are cylindrical and expandable from a small diameter to facilitate insertion through a small access. In general such stents have the openings in the walls to enhance overgrowth and ingrowth of tissue. Therefore the removal of expanded stents designed for permanent implantation is difficult and risky.

The golden standard for the treatment of benign prostate hyperplasia, BPH is transurethral resection TURP, which is surgery and means several days of hospitalisation and often results in severe side effects. It also means carrying an indwelling catheter during weeks and sometimes more.

Thermotherapy, such as heat treatment of the prostate with microwaves, interstitial radio frequency and hot balloons are interesting alternatives to TURP because it is less invasive and have less side effects. It is accomplished by heating the prostate to temperatures above 50-60°, which leads to decrease in the swelling of the prostate, as the prostatic gland.

A great problem connected to different kind of thermotherapy is however that the prostatic gland temporary also swells in response to the burn so that at higher temperatures and more efficient treatment there is a great risk for acute blockage of the urethra. A similar reaction happens at irradiation of prostatic cancer.

During the healing process of the prostate and the urethra the damaged tissue will partly be reabsorbed and partly slough off. The healing takes several weeks and because of the acute swelling it is therefore necessary to catheterise the patient during several weeks, which is a drawback to this kind of therapy. A long catheterisation time means not only a discomfort for the patient but also a risk for infections. Thermotherapy will therefore hardly replace TURP unless the catheterisation can be reduced to a few days or totally replaced.

It is therefore a need for a temporary stent to keep the urethra open allowing the patient to urinate during the healing. Such a stent must have an appropriate length and be precisely placed between the bladder neck and the outer sphincter to eliminate the risk for blockage by the swelling prostate on unsupported parts of the urethra or incontinence if a part of the stent protrudes into the sphincter. Such a temporary stent must also be easy to take out after the healing or in case of dislocation.

A commonly used material for stents is a so called memory or recovery metal such as Nitinol^{®}, which is an alloy based on nickel and titanium. Such alloys undergo a transition between a strong austenitic state and a soft martensitic state at certain temperatures. They have been subject to a great deal of interest due to the extraordinary "memory" they possess.

An expanded memorized shape can be set into such recovery metals by heating them while they are constrained in the desired expanded configuration. The forming temperature for setting the initial austenitic shape is typically around 500°C. After cooling the alloy to its martensite state it can be mechanically deformed to a second, smaller configuration, which is suitable for introduction in the organ to be stented. After placement the alloy is heated to its transition temperature, and expands to its austenite, strong state and recover its initial memorized configuration.

For replacement of indwelling catheters in urology particularily for the treatment of BPH, a stainless steel coil was introduced by Fabian 1980 followed by several similar stents for temporary use such as Prostakath™. A drawback with this type of non-expandable stents is that they often migrate.

Memokath^{®}, Engineers and Doctors, Denmark was developed to overcome the shortcomings of Prostakath™. It is a cylindrical coil stent made up of a single wire of a Nitinol^{®} alloy. The material has a transition temperature of about 45°C and becomes soft at 10°C. Because of the memory capability the stent is given a primary shape where one or more segments has a diameter which is considerably larger than the rest of the stent and a secondary shape where the segment with the larger diameter has been reduced to the same smaller diameter as the rest of the stent, WO 93/13824 and "From Prostakath® to Memokath®", Nordling et al. in "Stenting the Urinary System", pp. 285-290, Oxford Isis Medical Medica Ltd, 1998.

In the commercial configuration the Memokath^{®} stent has in its cylindrical non-expanded secondary shape an inner diameter of 6.7 mm and an outer diameter of 8.0 mm. In the primary shape is only the proximal part adjacent the sphincter shape expanded to a bell shape with tightly coiled wires. The expansion is activated by the injection of water at 50 to 60 °C, expanding the proximal part to an outer diameter of about 13 mm locking the stent in the urethra. The expansion is fast and starts by the unwinding of the proximal coil end and then coil by coil successively until the expansion is accomplished. The expansion to 13 mm is only for locking the stent to reduce the risk for migration. The inner diameter of 6.7 mm of the cylindrical part of the stent is sufficient to obtain a good flow.

The Memokath^{®} stent mainly used as a stent for long term placement can be taken out by using an endoscope with graspers. The endoscope has to be introduced via the meatus through the external sphincter. Ice-water is then injected softening the stent material allowing the stent coil to be partly stretched. By grasping the proximal stent end inside the sphincter, which sometimes can be difficult, can the stent then be taken out as a partly stretched wire by pulling the endoscope.

There is another stent, Horizon™ based on Nitinol for implantation in the prostatic urethra and where the fixation also is based on a bell shaped proximal part of the stent with the larger opening adjacent to the sphincter. In this case the transition temperature is low, about 37 °C, and the expansion is performed by the heat from the body.

Stents like Memokath™, Horizon™ have less tendency to migrate.compared to non-expanding stents if they are implanted to open up obstructions in the urethra. Clinical trials have showed however that such stents are not suitable to use as replacement for an indwelling catheter after thermo-therapy such as the Wallterm™ procedure as they show high migration rates of the stents into the bladder.

A reason seems to be that the thermal treatment first causes the urethra to widen before the prostatic gland starts to swell and thereby shrinks the urethra, which can create obstruction of the same type as described earlier.

If a stent of for ex type Memokath™ or Horizon™ is implanted in the widened urethra will the expanded, large bell shaped opening create strong outward forces of the sphincteric tissue counteracting the closing of it. If the urethra is widened the non-expanded part becomes loose and can be rejected into the bladder by the movement of the sphincter.

A typical stent with an introducer is disclosed in US-A-5540701.

It is thus an object of the present invention to produce a stent which can be precisely implanted in a diseased organ and have a low risk form migration, which is easy to implant, and to later remove in the case of a temporary stent, and which in general at least to some extent overcomes the disadvantages described above in relation to known stents.

Throughout this application a diseased organ may be e.g. an oesophagus being compressed due to a tumour, or obstructions in the urethra caused by BPH, temporary swelling of the prostatic gland after thermotherapy, irradiation or strictures.

### Summary of the invention

The invention is defined by claims 1 and 8.

The above object is achieved by means of a stent for use in a tubular organ of a body, comprising a first part to be positioned in a diseased part of the organ and acting as a stentbody therein, and a second part to be positioned in a non-diseased part of the organ and acting as an anchoring element, said two parts being designed as tubular members interconnected at the ends facing one another by a flexible, axially rigid connecting part, at least said second part having memorized therein a capacity to expand radially after an increase in temperature, thereby providing anchorage of the stentbody at a determined distance away from the diseased part of the organ. What is achieved is a stent which has a distinct stenting part, the first part, to keep the diseased organ open, and a distinct anchoring element, the second part, to anchor the stent in place in the non-diseased part. This anchoring works against migrations in both axial directions of the stent. The axially rigid connecting part is also serving this purpose of keeping the first part away from the surrounding organs, since the axial rigidity thereof transfers the anchoring from the second part to the first part. This way the first part is kept in position and is prohibited from migrating in either direction. However, the connecting part is not rigid in the transversal direction since it may need to follow the axial direction if the organ is not straight, or the movements of the organ.

Furthermore, by separating the stenting function and the anchoring function, each part may be thoroughly adapted to the circumstances under which it operates, i.e. each part may be adapted to restrictions in diseased or non-diseased parts or organs. For instance may the first part be given a diameter suitable to keep the diseased organ open, and the second part given another diameter for the anchoring function. A non-diseased part is in general less sensitive to extraordinary circumstances in comparison to a diseased part. Still, the unexpanded stent may be given the same features such as diameter all along.

The stent may be used in the urethra, wherein said first part is adapted to be positioned in the prostatic urethra, and said second part is adapted to be positioned in the bulbar urethra on the opposite side of a sphincter, wherein said connecting part has a length equal to or larger than the axial extension of the sphincter. What is achieved is a stent which has a distinct stenting part, the first part, to keep the diseased prostatic urethra open to allow a good urine flow, and a distinct anchoring element, the second part, to anchor the stent in place. This anchoring works in two directions, both anchoring against migration of the first part into the bladder, but also against migration towards the sphincter. The axially rigid connecting part is also serving the latter purpose, since the axial rigidity thereof transfers the anchoring of the second part to the first part. The anchoring against and the hindrance of migration towards the sphincter of the stent is an advantage since any hindrance of the normal movements of the sphincter may be inconvenient for the patient.

At least said second part is in its non-expanded state designed as a wound tubular spiral. The second part may thus easily and cheaply be produced and also easily made expandable.

In the expanded state of said stent, said second part at least at one portion along its axial length has a diameter that is larger than the diameter of the first part. In general, a diseased part is more vulnerable for irritation than a non-diseased part, and even though the stent is implanted in a patient for treatment of a disease, it is normally advantageous for the diameter of the first part to be smaller than of at least portions of the second part. Further, a diseased and treated part should not be expanded further, it should be kept open for a good urine flow, but it has been found that anchoring may still be achieved in non-diseased parts. In the expanded state of said stent, the second part at least at one portion along its axial length has a diameter that is at least 1.5 times the diameter of the first part. In certain organs, such as in the urethra when the stent is implanted on opposite sides of the sphincter, the non-diseased part may also under normal conditions be somewhat larger in diameter than the part which is to be treated. The bulbar urethra is, as the name implies, wider than the prostatic urethra, and it is thus advantageous to expand the second part to a diameter that is at least 1.5 times as large as the first part.

Said second part has a proximal end section and a distal end section, wherein said capacity to expand radially is memorised into at least one of said end sections.

Each axial expansion of said end sections forms a flaring flange.

Each flaring flange is facing away from the second part. Thus the second part is securely anchoring the stent in both axial direction.

Said capacity to expand extends axially all along the stent, whereby the whole stent may be expanded and given a suitable diameter in relation to both the diseased and the non-diseased parts. Especially if the pathway into the diseased part is tight, it may be painful for the patient unless the stent is made as small as possible until it is expanded to its treatment diameter.

A distal end of said second part is adapted to expand radially at expansion so as to be released from a catheter onto which the stent is mounted at implantation thereof into a patient. When the distal end of the second part, by the expansion of the stent, is expanding radially, the distal end is given a somewhat larger diameter than the catheter and catheter shaft. Also, the diameter of the second part becomes somewhat larger than a stop ring on the catheter that otherwise would keep the second part in place. Thus the second part may be passed over the stop ring and the catheter may be removed from the urethra.

At least said second part is made of a memory or a recovery metal. This way the capacity to expand is easily incorporated into the stent.

At least a proximal end of said second part is designed so as not to rotate during expansion thereof.

A thread is attached to the proximal end of said stent. The thread may be used to facilitate the removal of the stent by pulling it or by stretching it to prevent displacement of the stent when inserting an endoscope there through for inspection. To use ordinary graspers to grasp the wire end for removal of the stent is difficult and there is a risk that the wire end protrudes and causes damage to the patient at removal. Since the proximal end is not rotating, the thread will not be wound around the catheter shaft. Furthermore, when the thread is attached to the proximal end of the stent, the end of the stent will place itself in the longitudinal direction of the urethra when the thread is pulled for removal of the stent from the organ, the risk of scratching the organ will be reduced.

Said second part is divided into an even number of pairwise arranged sections wound in opposite directions, the change in direction between adjoining two sections being obtained by a turn or fold, said sections being of substantially equal length. This way, not only the distal end of the second part will stay fixed during the expansion as it is kept by the catheter, but also the proximal end, while the expansion takes place by rotation of the central part. Another advantage is that each rotating section, between the turns or folds, becomes shorter than if the second part would have been one long section. During rotation of one such section, of either length, the material of each section generates friction against the underlying catheter and the surrounding urethra. The shorter each section, the less friction is thus generated against the catheter, and the easier it is to expand the stent and later remove the catheter from the stent and urethra.

Its three parts are integrally formed in order to make the stent easy and cheap to produce.

According to the invention a catheter is disclosed for the implantation in a tubular organ of the body of a stent having a first part acting as a stentbody, a second part acting as an anchoring element and there between a connecting part, said catheter comprising a catheter shaft with a handle and stent expansion means at its proximal end and a stent holding part at its distal end, wherein said stent holding part is divided into a distal stentbody holding part, a proximal anchoring element holding part and there between holding means for releasable holding of said connecting part of the stent when said stent is mounted on said catheter. The stent is firmly held by the stent holding part, by said holding means, which may be important since the organ is sometimes not only tight but also not straight, such as the urethra. Thus a flexible and gentle catheter and stent is of importance to the patient. The usability and easiness of mounting of the stent on the catheter and its release are likewise important for the production and for the operator.

According to the invention said holding means is constituted by a sleeve enclosing said catheter shaft and provided with an axially extending slit along its length, said slit accommodating said connecting part when a stent is arranged on said catheter. The slit enables easy mounting and release of the stent from the catheter.

According to an embodiment of the present inventive concept said stent expansion means comprise connecting means at the proximal end of the catheter shaft for the introduction of a warm liquid into channels within the wall of said catheter shaft, which channels are interconnected with outlet openings at the stent holding part for said warm liquid, wherein said outlet openings are provided at least at the anchoring element holding part. No extra parts are thus needed for the introduction of warm liquid into the urethra for the expansion of the stent at implantation.

According to an embodiment of the present inventive concept, it further comprises a positioning balloon attached at the distal end of said catheter shaft and connected via the catheter shaft to a source of a pressure medium for the purpose of expanding the balloon when positioned in the urinary bladder during implantation. The exact location of the bladder neck, and thus the adequate position for the stentbody at the bladder neck if so desired may be easily found.

According to an embodiment of the present inventive concept said stent expansion means comprise a cylinder having wire locking means in its distal end and a operating element at its proximal end, said cylinder and operating element being rotationally and concentrically arranged around said catheter at said handle, wherein said cylinder, when a stent is arranged on said catheter, is adapted to control the expansion of the stent. This way there is a further means for controlling the expansion of the stent provided on the catheter. It may for the operator be advantageous to exactly know how and where the stent is expanding and by this feature also the speed of expansion may be controlled.

According to an embodiment of the present inventive concept said holding means is provided with at least one inspection opening through which the position of the proximal end of the stentbody in relation to the sphincter can be accurately determined by an endoscope introduced through a passage in the catheter shaft in use of said catheter. An endoscope is a standard instrument kept at hand by each urologist.

According to an embodiment of the present inventive concept said inspection opening extends somewhat into the proximal end of the part of the catheter shaft embraced by the stentbody when a stent is arranged on said catheter. If needed, the endoscope may be used to reduce the flexibility of the distal end of the catheter during implantation.

According to an embodiment of the present inventive concept said catheter is adapted for implantation of a stent according to any one of the earlier disclosed embodiments of the present inventive concept.

According to an embodiment of the present inventive concept, after expansion of said stent, said connecting part is arranged approximately at a centre line along the axial direction of said stent. Thus the connecting part is also provided approximately at the centre of the sphincter, causing less discomfort for the patient.

A method is disclosed for the implantation of a stent in a urethra using a catheter comprising a catheter shaft onto which a stent according to any one of the earlier disclosed aspects of the present invention is arranged, said method comprising the steps of: inserting the catheter carrying said stent into said urethra; positioning the catheter so as to place a stentbody of said stent within said urethra with an end thereof in close proximity of a sphincter; releasing the capacity to expand radially of at least an anchoring element of said stent; allowing at least said anchoring element to expand so as to provide anchorage of the stent in said position; and releasing said stent from said catheter shaft and withdrawing same from said urethra leaving said stent in position.

The capacity to expand is released by an increase in temperature caused by a warm liquid.

The capacity to expand is released by an increase in temperature caused by the body into which the catheter and stent is implanted. There is thus no need for any further step for releasing the capacity to expand.

The release of the stent from the catheter shaft is caused by the radial expansion of said anchoring element. There is thus no need for any further equipment or step for releasing the stent from the catheter.

According to an embodiment of the present inventive concept said catheter comprises in its distal end a balloon, wherein during positioning of the catheter with said stentbody within said prostate the distal end of the catheter is inserted into the bladder, said balloon is inflated within the bladder, and the catheter is retracted until the balloon is lying against the bladder neck, thereby defining the position of the stentbody at the bladder neck. A simple concept of implanting the stent may be used for the stent of the present inventive concept.

According to an embodiment of the present inventive concept said catheter is provided with at least one inspection opening enabling direct vision with an endoscope, wherein during positioning of the catheter with said stentbody within said prostate the inspection opening with the endoscope is inserted into the prostatic urethra; and finding via direct vision in the endoscope the distal end of the sphincter, thereby defining the position of the proximal part of the stentbody. Instead of as when implanting the stent with a balloon catheter implanting it by determining its position in relation to the bladder neck, it is in this embodiment possible to determine the position of the proximal part of the stentbody in relation to the distal part of the sphincter. If, in the balloon catheter positioning, the stentbody is chosen too long due to an incorrect measurement, the proximal end of the stentbody might end up into to the sphincter and cause incontinence. When instead using the above method, the accuracy of the measurement of the prostatic urethra is of less importance. It is fully acceptable if the distal end of the stentbody penetrates somewhat into the bladder compared to the consequences when the proximal end thereof penetrates into the sphincter. Another advantage is that by using this technique, the number of different stent-lengths to keep in stock may possibly be reduced to as few as two or three.

Said stent is made at least partly of a material having memory capacity, wherein said method further comprises the step of introducing a liquid at a temperature below a material softening point at the parts of the stent to be softened, thereby softening at least parts of the stent material. The stent, or the parts of the stent made of the memory capacity material, may thus be easily removed from the urethra. The operator is merely introducing the cooled liquid and softened parts may thus be withdrawn , almost in the shape and state of a long thread. If not softened, the stent would have to be removed in its expanded state, which could cause the patient some discomfort.

The liquid is introduced only into the bulbar urethra. This way the stent may be fully made of a material having memory capacity, but still only the part thereof that is situated within the bulbar urethra is made soft by said liquid. Thus the sphincter does not need to be penetrated as is necessary in prior art with less discomfort for the patient and easier handling for the operator as a result.

The method further comprises the step of grasping the proximal end of said stent and withdrawing said stent from the urethra.

The method further comprises the step of grasping a thread which is attached to the proximal end of said stent and withdrawing said stent from the urethra. It is simpler to grasp a thread which is lying slack within the urethra instead of trying to grasp the more rigid proximal end of the stent. The thread may have a length such that it almost ends up at the meatus of the penis, making it even simpler to grasp.

### Brief description of the enclosed drawings

Figure 1 is a partial longitudinal section of the region around the prostate of a patient having a known stent implanted there into,
Figure 2 is a partial longitudinal section of the region around the prostate of a patient having an unexpanded stent according to the present invention implanted,
Figure 3 partial longitudinal section of the region around the prostate having an expanded stent implanted,
Figure 4 is a partially cut, perspective view of a catheter to be used when implanting a stent according to the present invention,
Figure 5 is a cross section at A-A of figure 4 of the catheter,
Figure 6 is a side view of a catheter having mounted an unexpanded stent according to the present invention,
Figure 7 is a perspective view of a catheter having mounted an expanded stent,
Figure 8 is a side view of an anchoring element of a stent,
Figure 9 is a partial side view of the unexpanded anchoring element of figure 8 when mounted on a catheter,
Figure 10 is a perspective view of the expanded anchoring element of figure 8 and 9 when still mounted on the catheter,
Figure 11a - 11b are perspective views of different embodiments of the expanded anchoring element,
Figure 12 is a perspective view of the expanded anchoring element when still mounted on the catheter,
Figure 13a is a partial longitudinal section of the region around the prostate having an expanded stent according the figures 8 - 10 implanted,
Figure 13b is a partial longitudinal section of the region around the prostate having a stent implanted and the softened anchoring element partly removed there from,
Figure 13c is a partial longitudinal section of the region around the prostate when the softened anchoring element and the softened stentbody are partly removed there from by pulling the thread and the partially straightened wire,
Figure 14 is a partial longitudinal section of the region around the prostate having a stiff cylindrical stentbody implanted during its removal there from,
Figures 15a - 15d are side views of expanded anchoring elements of a stent,
Figures 15e - 15f are perspective views of expanded anchoring elements of a stent,
Figure 16 is a side view of an alternative embodiment of a catheter having mounted thereon an unexpanded stent according to the present invention with an endoscope,
Figure 17 is a cut perspective view of the catheter and stent according to figure 16,
Figure 18 is a cut side wide view of an alternative embodiment of a catheter having mounted thereon an unexpanded stent, and
Figure 19 is a side view of an alternative example of the anchoring element of a stent for use in relation to the catheter in figure 18.

### Detailed description of the drawings

In the claims and throughout this description the term axial direction is meant to be understood as a direction along the length of the urethra of a patient, and at the same time as a direction along the extension of a stent, between its proximal and distal ends, which is to be implanted within said urethra. The terms proximal and distal ends respectively are similarly meant to be understood as seen from the view point of the operator.

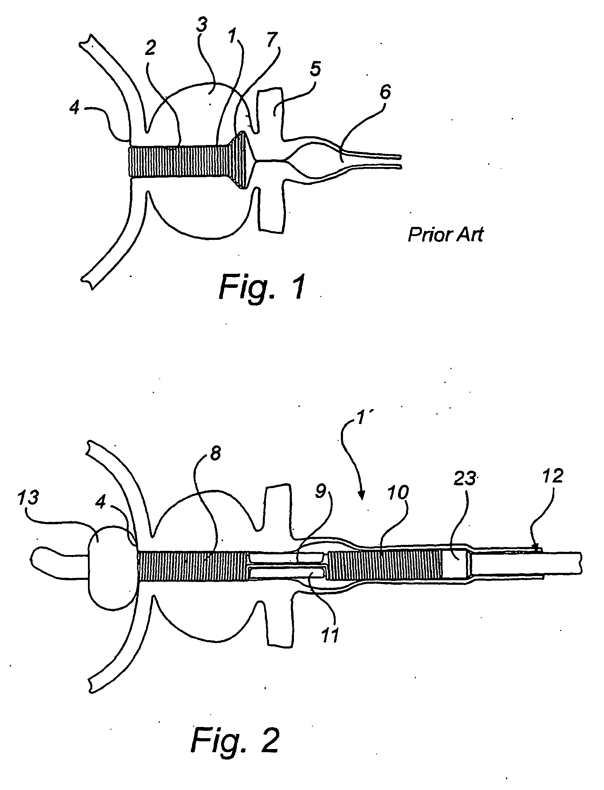

Figure 1 shows a Memokath® stent implanted in the prostatic urethra 2. The prostate is depicted 3, the bladder neck 4, the sphincter 5 and the bulbar urethra 6. The cylindrical part of the stent is depicted 1 and the expanded, bell shaped end 7 is adjacent to the sphincter 5. The purpose of the bell-shaped end is to lock the stent in the urethra and reduce the risk for migration axially, particularly into the sphincter 5. As thermotherapy temporary widens the prostatic urethra there is however also an increased risk for migration of the stent against the bladder with risk for acute retention of the urine. Furthermore the bell shaped end 7 close to the sphincter may cause problems as described earlier.

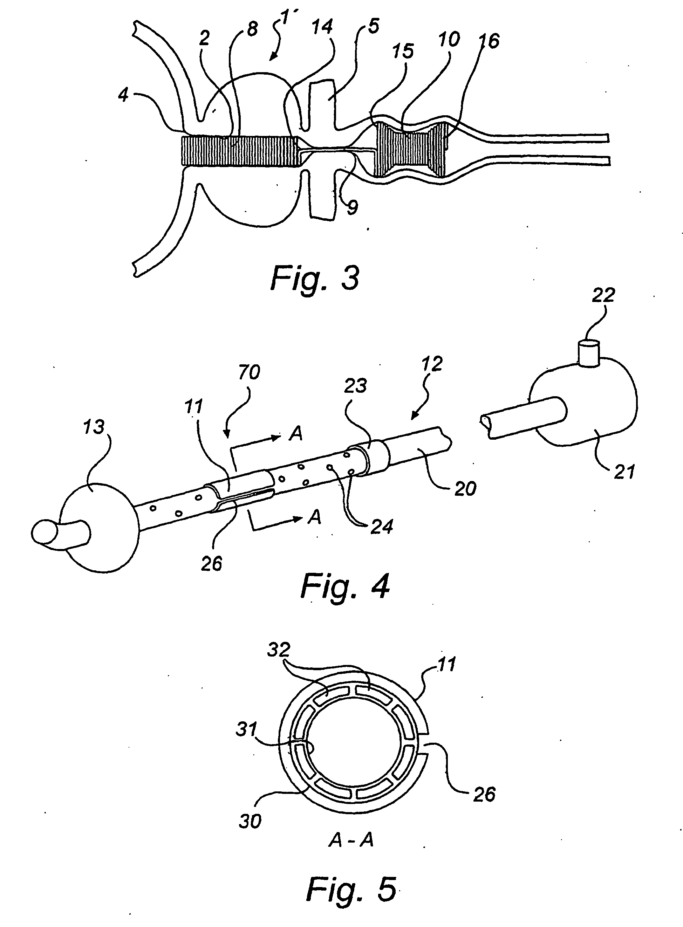

Figure 2 and 3 show a stent for a treatment of BPH. It may for instance be used as a temporary stent after thermotherapy or irradiation.

Figure 2 shows the proximal part of an introduction catheter 12 positioned for the placement of a stent in the urethra. The stent is composed of three elements: a cylindrical stentbody 8, a cylindrical but expandable anchoring element 10, and a connecting part 9 connecting the cylindrical stentbody 8 and the anchoring element 10. The connecting part 9 of the stent is flexible and detachably attached to the introduction catheter 12 with holding means 11, which will be described later. At the distal catheter tip a positioning balloon 13 is inflated. During the implantation procedure the catheter is slightly pulled backwards so that the balloon 13 pressures against the bladder neck 4. The stent has prior to its implantation been mounted on the shaft 12 between the positioning balloon 13 and a stop ring 23, and the length of the stentbody 8 has been selected so that it covers the desired length of the prostatic urethra. This selection is preferably made before the implantation of the stent by measuring the length of the prostatic urethra 2 with an endoscope. The stentbody 8, as well as the anchoring element 10 and the connecting part 9, could be made of the same memory material such as Nitinol, but the stentbody 8 and the connecting part 9 could also be made of different materials. It is however important that the anchoring element 10 is expandable for the anchoring of the stentbody 8. The stentbody 8 and the anchoring element 10 are designed as wound cylindrical spirals made of a wire of the memory metal Nitinol. When the stentbody 8, the anchoring element 10 and the connecting part 9 are all made of the same wire and thus of the same material, the production thereof will be particularly simple and thus cheap, and as will be demonstrated below, will be very easy to remove from the patient if so desired.

Figure 3 shows the stent after implantation, expansion and the removal of the catheter 12. The stentbody 8 is implanted into the prostatic urethra 2 from the bladder-neck 4 to a point 14 in the close proximity of the sphincter 5. Although there could be a desire to have a modest expansion of the stentbody 8 as well, there is thus no expanded part within the prostatic urethra 2 in the close proximity of the sphincter 5 for fixation of the stentbody 8, like in the case of the bell shaped proximal end of the known stent of figure 1. This could otherwise negatively affect the function of the sphincter 5. It is important that the stent does not hinder the function of the sphincter 5 by for instance preventing it from fully closing. If the sphincter 5 is prevented from working properly, the patient may suffer from urine leakage. The anchoring element 10 has been expanded by heating means, which will be shown later, in the catheter 12, and are forming two bell shaped flanges 15 and 16. The connecting part 9 has been detached from the holding means 11 during the expansion with means, which also will be described later.

The expanded form of the anchoring element 10, or of any one of the parts of the stent that should be expandable, has been given thereto by winding a Nitinol wire around a tool of the desired expanded configuration. The wound anchoring element 10 (or other part(s) to be expandable) in this way form a package. The package has then been heated at the forming temperature to set the initial shape as earlier described. After cooling to the martensite state the softened material has been wound around a shaft to the cylindrical configuration, as is shown in figure 2. When the stent possibly should be removed from the patient, only a limited number of parts of the stent, such as the anchoring element 10, may be cooled down to soften, even though more parts could be made of the material having memory capacity. The reason for this being that this could have advantages such as rational production in combination of the easy removal when limited to only one material.

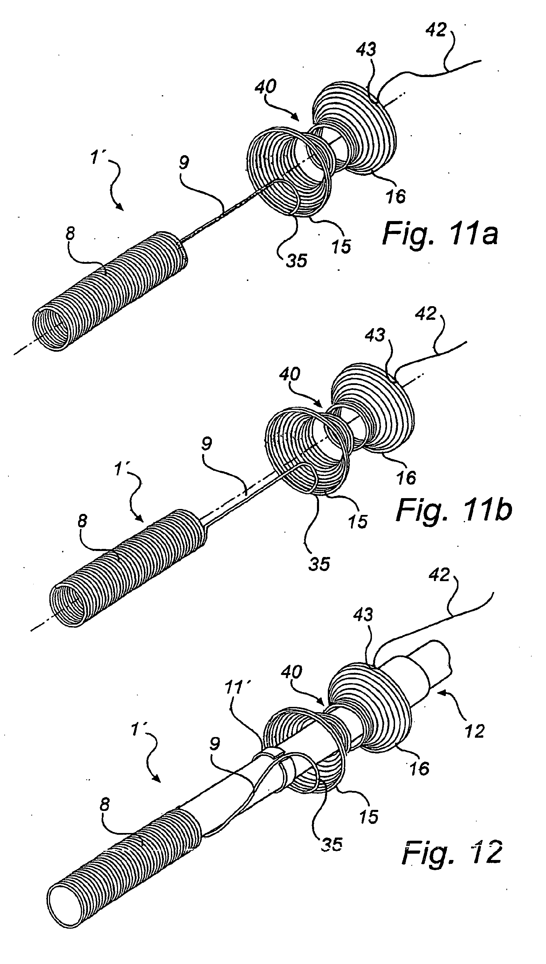

The connecting part 9 goes through the opening of the sphincter 5 without imparting the sphincter's 5 capability to fully close. The connecting part 9 is merely a wire around which the sphincter 5 can close and seal off the urethra at this point. Even though the connecting part 9 may be positioned not fully in the centre of the sphincter 5 when implanted in the body due to the manner in which the stent has been wound and memorised, the sphincter 5 still has the capacity to pinch and fully close the urethra. The connecting part 9 may for instance be somewhat flexible so that the closing of the sphincter 5 is pressing the connecting part 9 towards the centre of the sphincter 5 and thereby allowing symmetric closure of the sphincter 5. The connecting part 9 may also be preformed to get a curvature making it pass through the centre of the sphincter 5 and thereby reaching the same result. See further figures 11a - 11b and 12.

As shown in figure 2 the distal end of the anchoring element 10 is attached to the connecting part 9, which is detachably attached to the catheter 12 by the holding means 11. Therefore the anchoring element 10 expands by rotation of its proximal end. As the connecting part 9 is flexible but non compressible in the axial direction the distal flange 15 will as shown in figure 3 be expanded on a distance from the sphincter determined in combination by the preselected length of the connecting part 9, and by the positioning of the stentbody 8. The proximal flange 16 will in this embodiment of the invention move closer to the flange 15 as the anchoring element 10 shortens due to its expansion.

The anchoring element 10 with the flanges 15 and 16 have thus expanded in the bulbar urethra 6 which due to its tubular form is more suitable for anchoring than the diseased prostatic urethra 2. Another advantage is that the anchoring element 10 can be positioned on a preselected distance from the sphincter 5 avoiding interference with the sphincter's 5 movement. The double flanged anchoring element 10 also offers excellent resistance against migration of the stent in both of its axial directions in contrast to the prior art.

Figure 4 shows schematically the catheter 12 for positioning and implantation of the stent. The catheter shaft 20, a handle 21, connecting means 22 for introduction of hot liquid, the positioning balloon 13, holding means 11 at a stent holding part 70 for the stent 1' and the stop ring 23 both fixed to the catheter shaft and outlet holes 24 for hot water are depicted. The position of the outlet holes 24 is chosen so as not to distribute hot water into areas where it would only harm the tissue, or where the parts of the stent not having memory capacity are situated. The holding means 11 is attached to and around the catheter shaft 20 and is made of a tube with a longitudinal slit 26, along which slit 26 the connecting part 9 is positioned when the stent is arranged on the catheter 12, as shown in figure 2. The holding means 11 may be slipped on to the catheter 12 and could be using its inherent resiliency of the material to stay in place. Other means of arranging the holding means 11 onto the catheter 12 are gluing or welding it thereto. The thickness of the holding means 11 is approximately equal to the thickness of the wire of the stentbody 8. Figure 5 shows a cross section at A - A through the catheter shaft 20 in figure 4. The shaft 20 is flexible and has an outer wall 30 and an inner wall 31 at a distance from the outer wall 30, thus forming a multilumen with a number of longitudinally extending lumens 32. The lumens 32 are serving as channels for hot water for the expansion of the anchoring element 10. The water is injected through the means for introduction of hot water 22 (see figure 4) and will flow through connections to the channels 32 and out through the water outlets 24, shown in figure 4. In an other embodiment, one of the channels 32 can be used as a channel for injecting liquid for the inflation of the positioning balloon 13 in a known manner. In this embodiment the different channels 32 need to be separated from one another, at least those having different functions.

In order to be able to work efficiently and smoothly at implantation, it is desirable that the outer diameter of the stent is approximately 8 mm as a larger diameter can be painful for the patient. If the diameter it is very much smaller the flowrate will be reduced. It is generally contemplated that an expansion of approximately 1.5 - 2 times is the most that is recommended and conceivable. However, the stentbody 8 could be made somewhat smaller than 8 mm diameter at implantation in order to lessen the discomfort for the patient. If the stentbody 8 furthermore is made of a memory metal and is given an expanding feature, the stentbody 8 after implantation, at expansion, could expand up to the desired 8 mm diameter. The effect of increasing the diameter of the urethra is thus met.

With an unexpanded stentbody 8 of 8 mm diameter - and possibly also the whole unexpanded stent having a similar diameter, the wire is preferably approximately 0.65 mm in diameter and the inner diameter of the stentbody 8 thus approx. 6.7 mm. The outer diameter of the catheter suitably has an outer diameter of 6.3 mm leaving a clearance in relation to the stent of 0.2 mm on each side which is enough for the catheter to be easily removed from the stent, even if the stentbody 8 is not expanded. The inner diameter of the catheter is suitably 4.5 mm which will give a clearance of about 0.25 when using a standard endoscope of 4 mm in diameter.

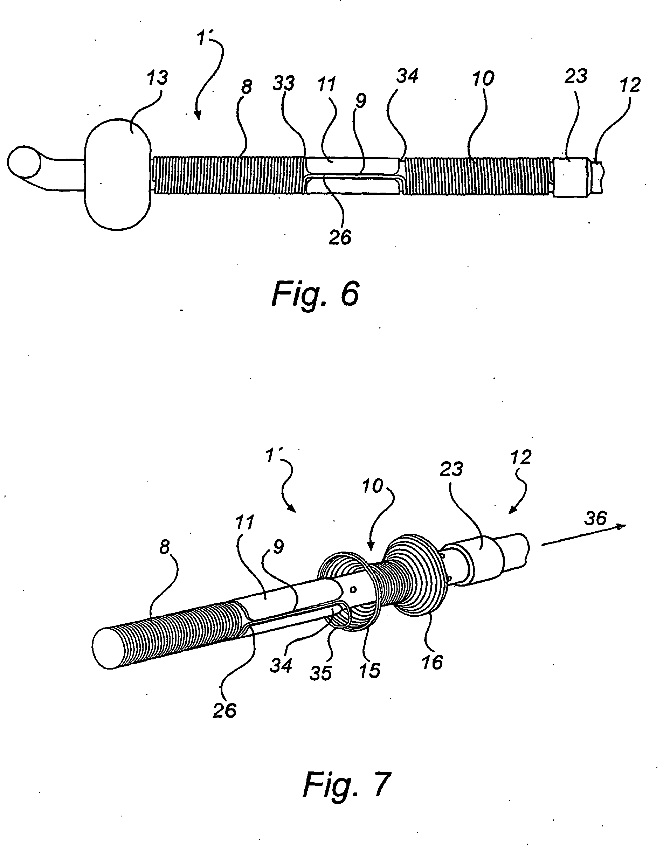

Figure 6 and 7 show the fixation and release after the implantation of the stent. Figure 6 shows in detail the stent arranged on the catheter and in position for implantation and corresponds to the situation according to figure 2, but for legibility the urethral organs are not shown.

As can be understood of earlier description the different parts of the stent are fixed in the axial direction, so for example is the stentbody 8 fixed between the balloon 13 and distal edge 33 of the tubular holding means 11, and the anchoring element 10 between the proximal edge 34 of the holding means 11 and the stop ring 23. Furthermore, the connecting part 9 is positioned in the slit 26 and therefore the mounted stent and its different elements can not rotate in relation to the catheter 12. This is particularly important during the introduction of the stent when manipulations in different directions are necessary in order to get the stent into place.

Figure 7 shows a part of the catheter 12, the stentbody 8, the connecting part 9 positioned in the slit 26, and its expanded anchoring element 10 with its bell shaped flanges 15 and 16 after the expansion. The figure shows how the first turn 35 of the expanded flange 15 at expansion releases the stop function which until this point has prevented the stent from being released from the catheter. This means that the first turn 35 of the wire which before expansion had been blocking the passing of the anchoring element 10 now can pass over the holding means 11. The reason is that at expansion the first turn 35 is rotating the connection part 9 approximately 90 degrees at the end 34 close to the holding means 11, since the diameter of the first turn 35 now is much larger than initially. At the same time the mid section of the anchoring element 10 between the two bell shaped flanges 15 and 16 is also expands somewhat. Not as much as the flanges 15 and 16, but enough to get a diameter that can be passed by on the outside of the holding means 11 and the stop ring 23, thus allowing the catheter 12 to be the pulled out axially from the urethra in direction of the arrow 36.

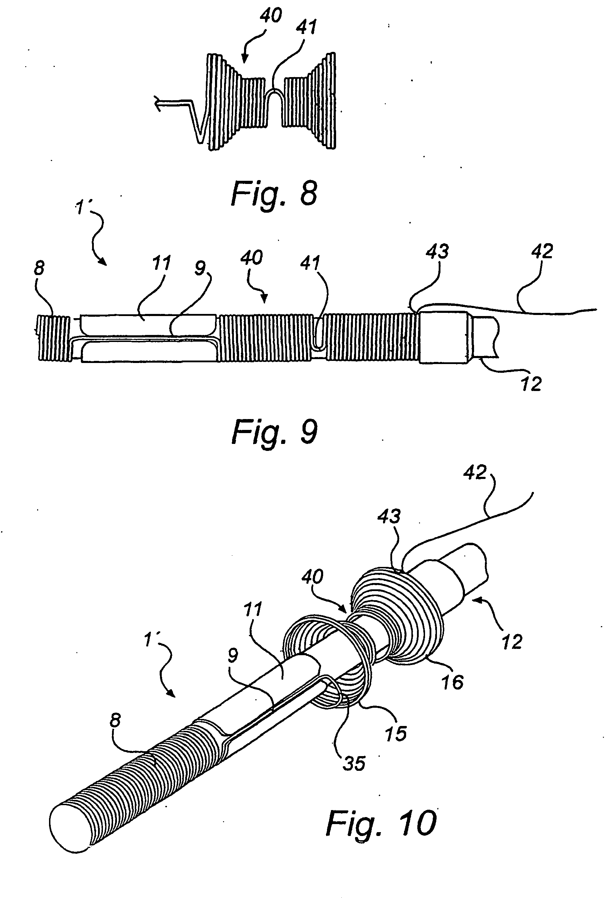

Figure 8 shows an expanded anchoring element 40 with two flanges. The two halves of the twin anchor is wound in counter directions so that the change of direction is creating a turn depicted 41, preferably in the middle of the anchoring element, making the anchoring element 40 symmetrical as seen in the axial direction.

Figure 9 shows a detail of the example according to figure 8 mounted on a catheter 12 with a part of a stentbody 8 detachably attached to the catheter by the holding means 11, the connecting part 9 and the turn 41. A thin thread 42 of suture type is attached to the wire end 43.

Figure 10 shows the example according to figure 8 and 9, but after expansion of the anchoring element 40. The catheter 12 according to the invention will be released at expansion as already described in connection to figure 7. At expansion the connecting part 9 is still kept in its slot preventing the distal end 15 of the flange to rotate but allowing expansion. The centre part with turn 41 will however rotate under expansion while its wire end 43 and attached thread 42 will radially expand without rotation.

As the end of the wire 43 will stay fix, the thread 42 will not be wound around the catheter at expansion as it would have been if a similar thread had been attached to the corresponding wire end in the example according to figure 7. If the thread 42 turns around the catheter's shaft there would be a risk that the stent and the anchoring element 40 would be pulled out with the catheter 12.

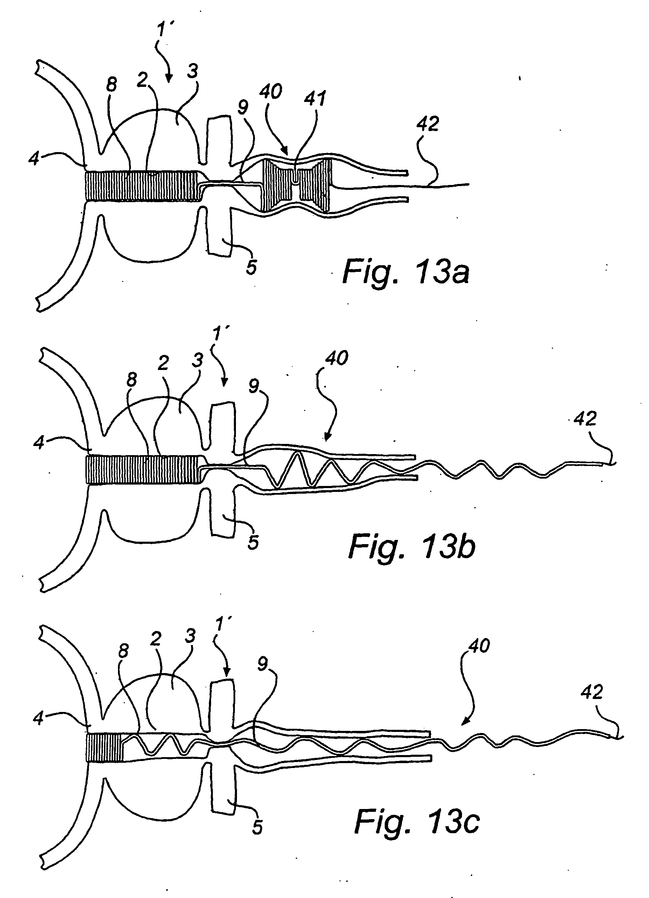

The possibility to attach a thread to the proximal part of the anchoring element 40 has many advantages. For example can a long thread 42 be left after implantation floating in the bulbar urethra with the end close to the meatus. After injection of cooled down water as described earlier the end of the thread may easily be grasped and the softened stent be pulled out. See in particular figures 13a - 13c.

Figures 11a and 11b illustrate different examples of the stent in which the connecting parts 9 have been differently designed when compared to one another. In both figures the expanded stent has been left in the urethra and the catheter is withdrawn. Also, both stents are made in one piece of one wire of memory metal. The parts of the wire making up the connecting parts 9, and approximately half a turn on each side thereof, i.e. half a turn at the proximal parts of the anchoring element 10 and half a turn of the distal parts of the stent bodies 8, have been ground to become somewhat thinner in diameter. Thereby the axial stiffness is maintained while the bending stiffness is lessened. In the example illustrated in figure 11a, the memory capacity has been utilised to give the connecting part 9 a position in relation to the stent 1' somewhat to the side of the centre line, which is illustrated by a dashed and dotted line. The axial stiffness is maintaining the function of the anchoring of the stent 1', but the reduced bending stiffness of connecting part 9 makes it easier for the sphincter 5 to position the connecting part 9 in the approximate centre of the urethra 6, thereby reducing the risk for discomfort for the patient. In this example the connecting part 9 will rebound to its original expanded position when the sphincter 5 opens, and thus the connecting part 9 will leave way for instruments penetrating the sphincter 5. In figure 11b the connecting part 9 is instead given an expanded shape in which it is positioned in or near the centre line of the stent 1'. This example enables the sphincter 5 to close in its centre. When an instrument, such as an endoscope, is used the connecting part 9 is moved to the side.

Figure 12 illustrates yet another example of the stent in which the connecting part 9 has been given an expanded shape which is curved and approximately follows the envelope surface of the catheter shaft. Thereby the holding element 11' is shorter in axial extension along the catheter to accommodate the connecting part 9. Also in this example the connecting part 9 is easier brought into the centre of the sphincter 5 when closing.

Figures 13a -13c also illustrate one example of the present invention, implanted within the prostatic urethra 2, in which the whole stent is made of a material with memory capability. The stentbody 8, the connecting part 9 and the anchoring element 10 are thus made integrally in one piece of a memory material such as Nitinol. The stent will assume the martensitic state when cold water is flowing over it. This means that an operator has to inject cold water not only into the bulbar urethra, but also to pass the sphincter 5 to let the water flow within the prostatic urethra 2. After the stentbody 8, the connecting part 9 and the anchoring element 10 have been treated with cold water to soften, they will in general keep their initial state until the operator starts pulling them out. This is shown in Figure 13a. The anchoring element 10 first starts to unwind and straighten out as shown in Figure 13b. When a part of the anchoring element 10 is unwound, the force on the stentbody 8, via the connecting part 9, is becoming so high that also the stentbody 8 unwinds, see figure 13c. When the stentbody 8 is unwound it is easy to remove from the prostatic urethra 2.

Another advantage by using a thread 42 is that by grasping the thread 42 the stent can be held so that a Foley catheter can be inserted through the stent in case of acute retention eliminating the risk for migration by pushing the anchoring element 10 and the stentbody 8.

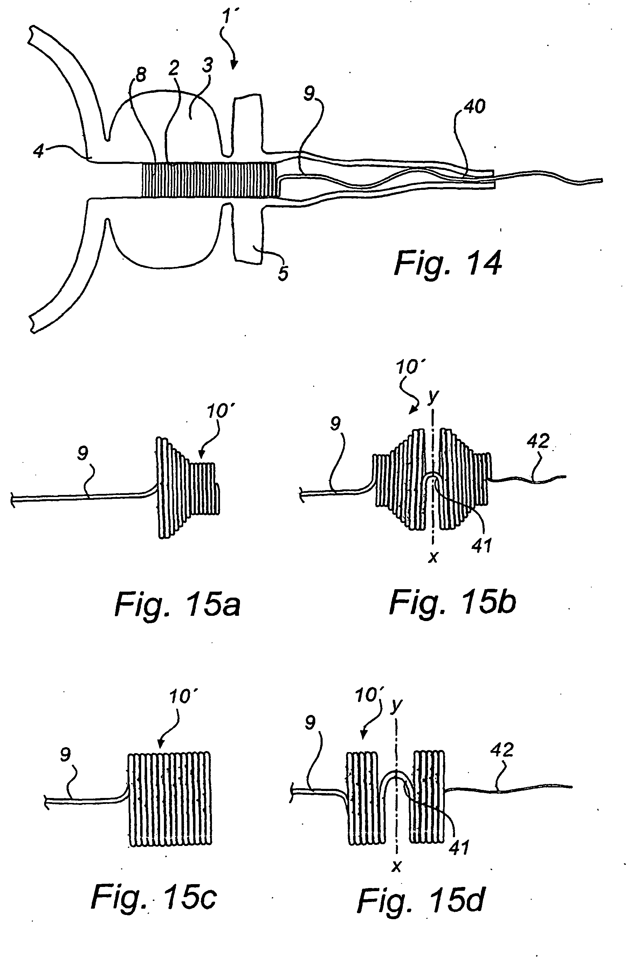

Figure 14 also shows a stent during its removal from the patient, after the anchoring element has been cooled down to soften. The stentbody 8 of the stent in this representation of the invention has either been produced from a material not having memory capability such as a spiral of a polymer or a cylindrical stentbody of an other design and material, or has not been cooled down to soften even though it has been made of a material having memory capability such as Nitinol, or has been differently heat treated so it will not soften at the same temperature as the anchoring element 10. In these three embodiments, only a small amount of coldwater has to be injected in the bulbar urethra 6 and there is no need to penetrate the external sphincter to soften the stentbody 8. This is an advantage of these three examples, since it may cause the patient some discomfort to pass the sphincter 5 with an instrument to flow the cold water within the prostatic urethra 2. The procedure is thus very simple and can easily be performed with a simple syringe by a nurse. The anchoring element 10 is however in these three examples of figure 14 made of a material having memory capability and will be softened by the cold water treatment, and this part will first be partly straightened out before the force withdrawing the stent forces the stentbody 8 pass the sphincter 5. The stentbody 8 is thus passing the sphincter 5 in its initial state. As mentioned, the stentbody 8 in these three embodiments does not need to be made in the form of a tubular spiral, but may have any tubular configuration such as a braided structure.

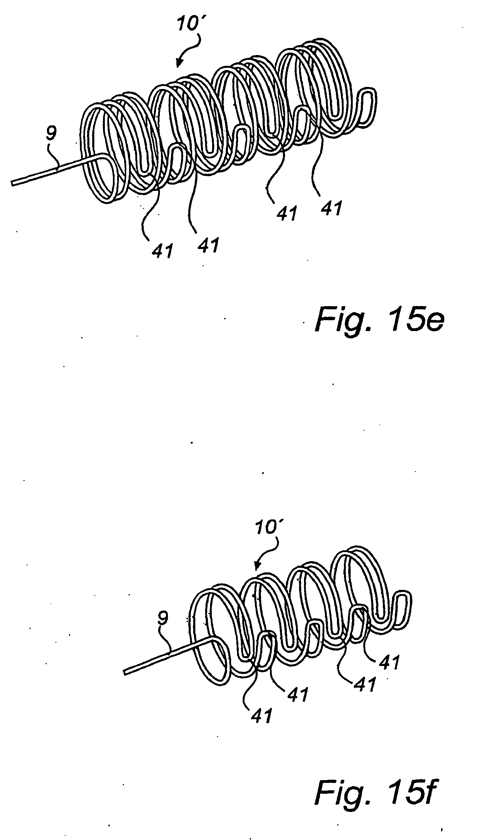

Figure 15 a-f show some alternative examples of expanded anchoring elements 10' within the scope of the invention. In figure 15b and 15d anchoring elements 10' are shown which are wound so that the proximal wire end will not rotate at expansion, and a thread 42 can be attached as described in connection with figure 8. If a thread 42 would be attached to a rotating proximal end of the anchoring element 10' such as the examples of figures 15a and 15c, the thread 42 could be wound around the catheter 20 hindering it from being removed from the urethra. However a rather short thread 42 could be attached and still facilitate the grasping of the proximal end of the stent as long as it does not hinder the catheter 20 from withdrawing after a rotating expansion of the stent. Alternatives to achieve the non-rotating effect are possible under condition that the winding is equal on the both sides of a symmetrical line x-y as shown in figures 15b and 15d. Another example is shown in figure 15e is when four turns 41 is present in the anchoring element 10'. This example does not rotate in its proximal end of the anchoring element 10', but individual parts thereof does. Yet another example which does has a non-rotating proximal end is shown in figure 15f. This anchoring element 10' is not rotating at expansion, but merely opening by dilating the turns 41' away from each other. There is thus no fully wound spiral in this example.

In order for the stent to be released from the catheter, the whole anchoring element 10, 40 and 10' must at least expand somewhat. Otherwise it may not be passed over the stop ring 23.

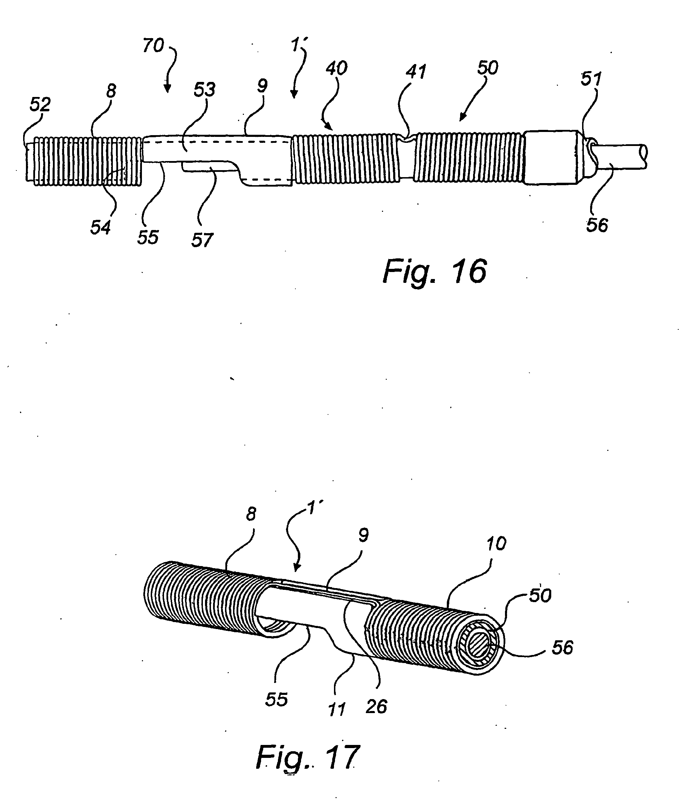

As mentioned it is desirable that the stentbody 8 at implantation covers as much as possible of the prostatic urethra 2 from the bladder-neck 4 to the sphincter 5 without disturbing the function of the sphincter 5. Figure 16 shows an embodiment of a catheter which allows a precise placement of the stentbody 8 in the prostatic urethra 2 under direct vision with an endoscope. In this embodiment there is no use of a positioning balloon 13 as described in relation to figures 4 and 6.

Figure 16 is a side view of the distal part of a catheter 50 with the same means for the fixation of a stentbody 8 and of the anchoring means 10 respectively, as described in relation to figures 4 and 6. The catheter shaft consists of a proximal part 51 in the form of a flexible multilumen tube for injection of hot water as earlier described. The lumens extend to the distal border of the anchoring means 10 and the rest of the catheter shaft continues to the catheter tip 52 in the form of a tube with a solid but flexible wall of the same inner and outer diameter as the multilumen tube, i.e. it is in this part solid.

In the catheter shaft an opening 54 is arranged (broken line) together with an opening 55 in the holding means 53 creating an opening extending at least to the distal end of the holding means 53. An endoscope or telescope 56 is positioned within the catheter shaft 51 and can be moved axially along the shaft 51.

In figure 16 the tip 57 of the endoscope 56 is positioned in the opening 55, allowing a precise control of the position of the proximal first turns of the stentbody 8 in relation to the sphincter 5. By advancing the endoscope 56 the position of the distal part of the stentbody 8 can also be controlled. If a correct length of the stentbody 8 has been selected by the operator, the stentbody 8 should extend along the desired length of the prostatic urethra 2, which in most cases if from the bladder-neck 4 over the very montanum to a position in the close proximity of the sphincter 8.

An advantage with this method in contrast to the method of positioning the stent starting from the bladder-neck 4 using a positioning balloon 13, is that the operator can select a stentbody 8 which is somewhat longer than the measured needed length between the bladder-neck 4 and the sphincter 5. If there is an excess in length of the stentbody 8 after the precise placement going out from the sphincter 5 side, this excess will protrude somewhat into the bladder which will not harm.

Alternatively, the catheter shaft 51 inside the stentbody 8, can be made very short. This way it will only protrude a very short distance into the stentbody 8, possibly only a few millimetres. The most part of the stentbody 8 is thus not supported by the catheter shaft 51 and will be very flexible. In order to be able to implant the stent, the endoscope however will have to be pushed forwards towards the distal part of the stentbody 8 for keeping it supported, at least before and during the implantation.

Figure 17 shows in perspective the stentbody 8, the connecting part 9 within the holding means, the anchoring element 10 and the opening 55 of the catheter which is also shown in figure 16.

It is suitable to produce stents having stentbodies 8 of different lengths in order for the operator to be able to chose an appropriate length for each patient. The stents to be implanted using a catheter with a positioning balloon 13 need preferably to come in 5 mm incremental stentbody 8 lengths, such as 25, 30, 35 mm etc. The stents to be implanted using a catheter with an endoscope 56 need however preferably to come in only 10 - 15 mm incremental stentbody 8 lengths, such as 40, 50, 60 mm. The difference between the two methods is that since the endoscopic method is going out from the position of the sphincter 5, the stent may penetrate somewhat into the bladder. However, in the balloon-method, the position of the bladder-neck 4 is decisive for the position of the stentbody 8, and if this is too long it will penetrate into the sphincter 5, which is negative for the sphincter performance. It should also be remembered that the initial measurement of the length of the prostatic urethra 2 is not exact, but involving a certain amount of uncertainty, making the choice of stentbody 8 length somewhat difficult to begin with. At least when the stent is used as a temporary stent, it is not harmful for the patient of the stentbody 8 penetrates into the bladder. Thus, when using the endoscopic method, the operator needs to have in store only half as many stents, or two to three different lengths, as if instead the balloon-method would be used, saving both money and storage capacity.

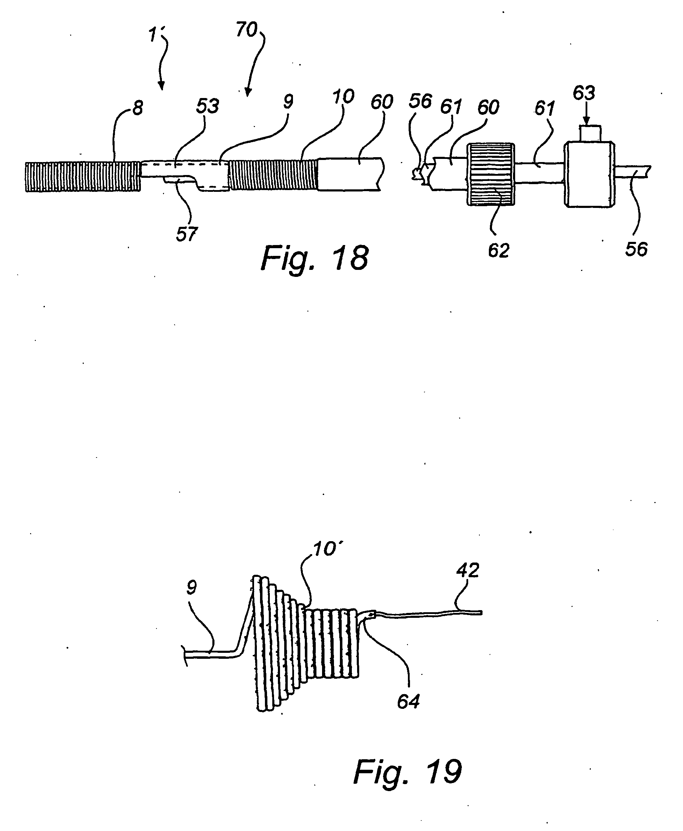

Figure 18 shows an alternative embodiment of a catheter and a mounted stent also allowing precise placement of the stent under direct vision. The stent 1' or at least the anchoring element 10 has preferably expanded configuration corresponding to figures 15a or 15c. The proximal part of the stent 1' is detachably attached to an outer rotatable flexible cylinder 60 concentric in relation to a catheter shaft 61, which corresponds to the shaft 51 and 52 shown in figure 16. The cylinder 60, is however solid without channels for injection of a warm liquid such as water. In this embodiment the anchoring means 10, and possibly the entire stent 1' is selected of a memory metal, such as Nitinol with different characteristics compared to the earlier described embodiments. So for example, could here a Nitinol material be used which has a superelastic and austenitic state at the body temperature of the patient, and therefore there is no need for the hot liquid for the expansion of the anchoring means 10.

At the mounting of the stent on the catheter before implantation, the anchoring element 10 which is in a soft martensitic state, has been attached to the cylinder 60 and stretched and been given its cylindrical shape by rotating the wheel 62 and then been locked in place to the catheter by locking means not shown.

A telescope 56 is in a well known manner concentrically arranged inside the cylinder 60 in order for it to be slidable axially. A space (not shown) is created between the telescope 56 and the cylinder 60, allowing rinsing water flow from the inlet 63 along the telescope to keep the lens of the telescope 56 clean.

The implantation of the stent in the urethra 2, 6 takes place as earlier described in connection with figure 16. There are however some minor differences between the method of implantation of this latter stent and the already described one in relation to figure 16. Due to the influence of the body temperature the anchoring means 10 (or alternatively the whole stent 1') will not before entering the body change into a austenitic state. It cannot however take its memorised shape as it is kept fixed in its both ends.

When the operator finds that the,stentbody 8 is correctly positioned he can let the anchoring means 10 expand by turning the wheel 62 in opposite direction to the earlier mounting direction of the wound spiral. The distal end of the anchoring means 10 will be released by the expansion from the holding means 53. The proximal end of the anchoring means 10 can also be released automatically because of the axial shortening of the anchoring means 10 when expanding. Figure 19 shows a modification of the anchoring means 10 in which the proximal end thereof is protruding 64 axially towards the operator. This protruding part 64 may in turn be provided with a thread of the same reasons as has earlier been described. The protruding part 64 may have a complimentary shaped slit in the distal end of the cylinder 60, into which slit the protruding part 64 can be placed so as to lock the rotation of the anchoring means 10 in relation to the cylinder 60.

The method of producing the stent according to the present invention may thus be summarised in the steps of winding a wire of a material having memory capability around a tool having the shape of the expanded stent. Thereafter the material is heated up to its forming temperature. Then the wire is cooled down to its martensite state and rewound to correspond to the unexpanded shape and in which it may be placed around a catheter 12 prepared for implantation. When the stent has been arranged on the catheter 12 it is ready to be implanted into the body by inserting it via the urethra.

The method of implanting the stent and catheter into the urethra in begun by measuring the length of the prostatic urethra 2, for instance with an endoscope and.a scale, in order to chose a stentbody 8 of a correct length. An anaesthetic agent is then spread in the urethra. Thereafter the implantation of the stent via the urethra 6 takes place. If the stent is implanted with the aid of a catheter according for example figure 4, the catheter 12 with the stent is inserted a short distance further into the urethra than required and in this position the balloon 13 is inflated via the channels 32. In this state the catheter 12 is pulled back towards the operator until the balloon 13 bears against the inside of the bladder. Now the stent is in place. If instead the stent is implanted with the aid of a catheter according to figure 16, the endoscope 56 is inserted within the catheter and the stent so that the lens of the endoscope is positioned at the distal end of the catheter 52 and then locked in relation to the catheter forming an assembly, which then is inserted into the urethra under continued flushing of the endoscope with water for it to be clean for vision until the distal end 52 protrudes somewhat into the bladder. The endoscope is released from the catheter and pulled backwards until the operator finds the opening 55 in the catheter. Through the opening 55 the operator can seek and find the distal end of the sphincter 5. The catheter with the stent is then positioned so that the proximal end of the stentbody 8 ends up in the close proximity of the sphincter 5. The stent is now in place. As there is a length of about 20 mm or more of the connecting means, the distal part of the anchoring element 10 will also be close to the sphincter 5. The stentbody 8 will in this position and after the later expansion be quite close to the sphincter 5 in order to securely keep the full prostatic urethra open. However, thanks to the extension of the connecting means 9, the anchoring element 10 is kept at a safe distance from the sphincter 5 at its proximal end.

In both of the above described alternatives of the method of placing the stent within the urethra, hot water is now injected into the channels 32 and out through the outlet holes 24. Under normal circumstances the hot water should be spread through the outlet holes 24 simultaneously. When the hot water meets the memory metal parts of the stent, especially the anchoring element 10, these parts within almost no time at all expand to its expanded state. Now the catheter is released and be pulled out with the endoscope, leaving the stent implanted. If the patient needs the be examined within the urethra or the bladder, or needs a Foley catheter to be implanted, such catheters or endoscopes may still be passed through the stent since it does not block the passage way into the bladder. The thread 42, could be stretched with a grasper to prevent that the stent will be pushed forwards.

When and if the stent should be removed again from the patient, ice-water is inserted into the urethra at the areas where the stent should be softened. The treating doctor or nurse may then grasp the thread 42 and simply and gently pull out the stent.

When using the stent and catheter of figure 18, the method of implanting the stent is somewhat different to what is described above in relation to figures 1 - 16. The major difference being that the material of the anchoring element 10 has been treated so it will have a transition temperature below 37 °C and preferably be in a superelastic state at body temperature. By mounting the stent on the catheter and implanting it, possibly with an endoscope, into the urethra, the transition temperature of the material is reached and the capacity to expand is thereby released. Due to the locking means, for instance the pin 64 inserted into the slit in the cylinder 60, the anchoring element 10 is however still prohibited from expanding. Until the operator releases the cylinder 60 by either self determining the rotation speed, or by fully releasing the cylinder 60, the capacity to expand of the anchoring element 10 forces the stent and the cylinder 60 to rotate in the expansion direction of the anchoring element 10. Now the stent is in place and the catheter and endoscope may be withdrawn. Any release and later withdrawal of the stent follows the earlier described procedure.

The catheters for the different embodiments of the inventive concept can be made very cheaply, particularly the one using the endoscopic method, and can therefore be used as disposables. Thus catheters with different stent body lengths can be mounted directly upon production. The operator consequently only has to select the suitable stent body length. He or she is thus released from the task to mount the stent on the catheter.

It is to be understood that various modifications, alterations and adaptations may be made by those skilled in the art without departing from scope of this invention. The manifestations of the form in the figures of the implant insertion devices according to the present invention are only schematic representations. For instance is it not necessary that the stent according to the present invention is a temporary stent, but it could also successfully be used as a permanent stent. Furthermore, the stent may be used not only in combination with thermotherapy of the prostate, but as a stent for keeping the prostatic urethra open with or without the preceding thermotherapy. Also, the stent may be used in the treatment of strictures in the bulbar urethra, in which case the anchoring element 10 instead would be implanted within the prostatic urethra 2 and the stentbody 8 would be implanted within the bulbar urethra 6. The stent may be used in any method of keeping a tubular organ of the body open for passage, such as the oesophagus.

## Claims

1. A catheter (12, 50) for the implantation in a tubular organ of the body of a stent (1') having a first part acting as a stentbody (8), a second part acting as an anchoring element (10, 10', 40) and there between a connecting part (9), said catheter (12, 50) comprising a catheter shaft (20, 61, 51) with a handle (21) and stent expansion means at its proximal end and a stent holding part (70) at its distal end, wherein said stent holding part (70) is divided into a distal stentbody holding part, a proximal anchoring element holding part and there between holding means (11, 53) for releasable holding of said connecting part (9) of the stent (1') when said stent (1') is arranged on said catheter (12, 50),
**characterised in that** said holding means (11, 53) is constituted by a sleeve enclosing said catheter shaft (20, 51, 52; 61) and provided with an axially extending slit (26) along its length, said slit (26) accommodating said connecting part (9) when a stent (1) is arranged on said catheter (12, 50).

2. A catheter (12) according to claim 1, wherein said stent expansion means comprise connecting means (22) at the proximal end of the catheter shaft (20) for the introduction of a warm liquid into channels (32) within the wall of said catheter shaft (20), which channels (32) are interconnected with outlet openings (24) at the stent holding part (70) for said warm liquid, wherein said outlet openings (24) are provided at least at the anchoring element holding part.

3. A catheter (12) according to any one of the claims 1 - 2, further comprising a positioning balloon (13) attached at the distal end of said catheter shaft (20) and connected via the catheter shaft (20) to a source of a pressure medium for the purpose of expanding the balloon (13) when positioned in the urinary bladder during implantation.

4. A catheter (50) according to claim 1, wherein said stent expansion means comprise a cylinder (60) having wire locking means in its distal end and an operating element (62) at its proximal end, said cylinder (60) and operating element (62) being rotationally and concentrically arranged around said catheter (50) at said handle (21), wherein said cylinder (60), when a stent (1') is arranged on said catheter (50), is adapted to control the expansion of the stent (1').

5. A catheter (12, 50) according to any one of the claims 1 or 4, wherein said holding means (11, 53) is provided with at least one inspection opening (55) through which the position of the proximal end of the stentbody (8) in relation to the sphincter (5) can be accurately determined by an endoscope (57) introduced through a passage in the catheter shaft (20, 51, 52; 61) in use of said catheter (12, 50).

6. A catheter (12, 50) according to claim 5, wherein said inspection opening (55) extends somewhat into the proximal end of the part of the catheter shaft (20, 51, 52; 61) embraced by the stentbody (8) when a stent (1') is arranged on said catheter (12, 50).

7. A system comprising a stent (1') and a catheter (12, 50), said stent (1') comprising a first part (8) to be positioned in a diseased part (2) of the organ and acting as a stentbody therein, and a second part (10, 10', 40) to be positioned in a non-diseased part (6) of the organ and acting as an anchoring element therein, said two parts (8; 10, 10', 40) being designed as tubular members interconnected at the ends facing one another buy a flexible, axially rigid connecting part (9), at least said second part (10, 10', 40) having memorised therein a capacity to expand radially after an increase in temperature, thereby providing anchorage of the first part (8) at a determined distance away from the first bodily part (2) of the organ, said catheter (12, 50) comprising a catheter shaft (20, 61, 51) with a handle (21) and stent expansion means at its proximal end and a stent holding part (70) at its distal end, wherein said stent holding part (70) is divided into a distal stentbody holding part, a proximal anchoring element holding part and there between holding means (11, 53) for releasable holding of said connecting part (9) of the stent (1') when said stent (1') is arranged on said catheter (12, 50), wherein said holding means (11, 53) is constituted by a sleeve enclosing said catheter shaft (20, 51, 52; 61) and provided with an axially extending slit (26) along its length, said slit (26) accommodating said connecting part (9) when a stent (1) is arranged on said catheter (12, 50)

8. A system according to claim 7 wherein said catheter (12, 50) is a catheter according to any one of claims 1 - 6.

## Patentansprüche

1. Katheter (12, 50) zur Implantation eines Stents (1') in ein röhrenförmiges Organ des Körpers, der einen als ein Stentkörper (8) agierenden ersten Teil, einen als ein Verankerungselement (10, 10', 40) agierenden zweiten Teil und dazwischen einen Verbindungsteil (9) aufweist, wobei der Katheter (12, 50) einen Katheterschaft (20, 61, 51) mit einem Griff (21) und Stent-Expansionsmittel an seinem proximalen Ende und einen Stent-Halteteil (70) an seinem distalen Ende umfasst, wobei der Stent-Halteteil (70) in einen distalen Stentkörper-Halteteil, einen proximalen Verankerungselement-Halteteil und dazwischen befindliche Haltemittel (11, 53) zum lösbaren Halten des Verbindungsteils (9) des Stents (1'), wenn der Stent (1') an dem Katheter (12, 50) angeordnet ist, unterteilt ist, **dadurch gekennzeichnet, dass** das Haltemittel (11, 53) von einer Hülse gebildet wird, die den Katheterschaft (20, 51, 52; 61) umschließt und mit einem sich axial erstreckenden Schlitz (26) entlang ihrer Länge versehen ist, wobei der Schlitz (26) den Verbindungsteil (9) aufnimmt, wenn ein Stent (1) an dem Katheter (12, 50) angeordnet ist.

2. Katheter (12) nach Anspruch 1, wobei die Stent-Expansionsmittel Verbindungsmittel (22) am proximalen Ende des Katheterschaftes (20) zur Einführung einer warmen Flüssigkeit in Kanäle (32) innerhalb der Wand des Katheterschaftes (20) umfassen, wobei die Kanäle (32) mit Auslassöffnungen (24) an dem Stent-Halteteil (70) für die warme Flüssigkeit verbunden sind, wobei die Auslassöffnungen (24) wenigstens an dem Verankerungselement-Halteteil vorgesehen sind.

3. Katheter (12) nach einem der Ansprüche 1-2, ferner einen Positionierungsballon (13) umfassend, der an dem distalen Ende des Katheterschaftes (20) befestigt ist und über den Katheterschaft (20) mit einer Quelle eines Druckmediums verbunden ist, zum Zwecke der Expansion des Ballons (13), wenn er während der Implantation in der Harnblase positioniert wird.

4. Katheter (50) nach Anspruch 1, wobei die Stent-Expansionsmittel einen Zylinder (60) mit Drahtverriegelungsmitteln in seinem distalen Ende und einem Betätigungselement (62) an seinem proximalen Ende umfassen, wobei der Zylinder (60) und das Betätigungselement (62) an dem Griff (21) drehbar und konzentrisch um den Katheter (50) herum angeordnet sind, wobei der Zylinder (60), wenn ein Stent (1') an dem Katheter (50) angeordnet ist, dazu eingerichtet ist, die Expansion des Stents (1') zu steuern.

5. Katheter (12, 50) nach einem der Ansprüche 1 oder 4, wobei das Haltemittel (11, 53) mit mindestens einer Inspektionsöffnung (55) versehen ist, durch welche hindurch die Position des proximalen Endes des Stentkörpers (8) in Bezug auf den Schließmuskel (5) durch ein Endoskop (57), das bei Verwendung des Katheters (12, 50) durch einen Durchgang in dem Katheterschaft (20, 51, 52; 61) eingeführt wird, genau bestimmt werden kann.

6. Katheter (12, 50) nach Anspruch 5, wobei sich die Inspektionsöffnung (55) etwas in das proximale Ende des von dem Stentkörper (8) umschlossenen Teils des Katheterschaftes (20, 51, 52; 61) hinein erstreckt, wenn ein Stent (1') an dem Katheter (12, 50) angeordnet ist.

7. System, welches einen Stent (1') und einen Katheter (12, 50) umfasst, wobei der Stent (1') einen ersten Teil (8), der in einem erkrankten Teil (2) des Organs zu positionieren ist und darin als ein Stentkörper agiert, und einen zweiten Teil (10, 10', 40), der in einem nicht erkrankten Teil (6) des Organs zu positionieren ist und darin als ein Verankerungselement agiert, umfasst, wobei die zwei Teile (8; 10, 10', 40) als rohrförmige Elemente ausgebildet sind, die an den einander zugewandten Enden durch einen flexiblen, axial starren Verbindungsteil (9) miteinander verbunden sind, wobei wenigstens in dem zweiten Teil (10, 10', 40) eine Fähigkeit gespeichert ist, nach einer Temperaturerhöhung radial zu expandieren, wodurch eine Verankerung des ersten Teils (8) in einer bestimmten Entfernung von dem ersten körperlichen Teil (2) des Organs gewährleistet wird, wobei der Katheter (12, 50) einen Katheterschaft (20, 61, 51) mit einem Griff (21) und Stent-Expansionsmittel an seinem proximalen Ende und einen Stent-Halteteil (70) an seinem distalen Ende umfasst, wobei der Stent-Halteteil (70) in einen distalen Stentkörper-Halteteil, einen proximalen Verankerungselement-Halteteil und dazwischen befindliche Haltemittel (11, 53) zum lösbaren Halten des Verbindungsteils (9) des Stents (1'), wenn der Stent (1') an dem Katheter (12, 50) angeordnet ist, unterteilt ist, wobei das Haltemittel (11, 53) von einer Hülse gebildet wird, die den Katheterschaft (20, 51, 52; 61) umschließt und mit einem sich axial erstreckenden Schlitz (26) entlang ihrer Länge versehen ist, wobei der Schlitz (26) den Verbindungsteil (9) aufnimmt, wenn ein Stent (1) an dem Katheter (12, 50) angeordnet ist.

8. System nach Anspruch 7, wobei der Katheter (12, 50) ein Katheter nach einem der Ansprüche 1-6 ist.

## Revendications

1. Cathéter (12, 50) pour l'implantation dans un organe tubulaire du corps d'un stent (1') ayant une première partie servant de corps de stent (8), une deuxième partie servant d'élément d'ancrage (10, 10', 40) et entre celles-ci une partie de connexion (9), ledit cathéter (12, 50) comprenant une tige de cathéter (20, 61, 51) avec une poignée (21) et un moyen de déploiement de stent au niveau de son extrémité proximale et une partie de retenue de stent (70) au niveau de son extrémité distale, ladite partie de retenue de stent (70) étant divisée en une partie de retenue de corps de stent distale, une partie de retenue d'élément d'ancrage proximale et entre celles-ci un moyen de retenue (11, 53) destiné à retenir de manière libérable ladite partie de connexion (9) du stent (1') lorsque ledit stent (1') est disposé sur ledit cathéter (12, 50), **caractérisé en ce que**l ledit moyen de retenue (11, 53) est constitué par un manchon entourant ladite tige de cathéter (20, 51, 52 ; 61) et doté d'une fente s'étendant axialement (26) sur sa longueur, ladite fente (26) accueillant ladite partie de connexion (9) lorsqu'un stent (1) est disposé sur ledit cathéter (12, 50).

2. Cathéter (12) selon la revendication 1, ledit moyen de déploiement de stent comprenant un moyen de connexion (22) au niveau de l'extrémité proximale de la tige de cathéter (20) pour l'introduction d'un liquide chaud dans les canaux (32) à l'intérieur de la paroi de ladite tige de cathéter (20), lesquels canaux (32) sont interconnectés avec des ouvertures de sortie (24) au niveau de la partie de retenue de stent (70) pour ledit liquide chaud, lesdites ouvertures de sortie (24) étant dotées d'au moins une partie de retenue d'élément d'ancrage.

3. Cathéter (12) selon l'une quelconque des revendications 1 et 2, comprenant en outre un ballonnet positionnable (13) fixé au niveau de l'extrémité distale de ladite tige de cathéter (20) et connecté via la tige de cathéter (20) à une source de milieu sous pression destiné à dilater le ballonnet (13) lorsqu'il est positionné dans la vessie durant l'implantation.

4. Cathéter (50) selon la revendication 1, ledit moyen de déploiement de stent comprenant un cylindre (60) ayant un moyen de blocage de fil dans son extrémité distale et un élément fonctionnel (62) au niveau de son extrémité proximale, ledit cylindre (60) et ledit élément fonctionnel (62) étant disposés de manière rotative et concentrique autour dudit cathéter (50) au niveau de ladite poignée (21), ledit cylindre (60), lorsqu'un stent (1') est disposé sur ledit cathéter (50), étant adapté pour contrôler le déploiement du stent (1').

5. Cathéter (12, 50) selon l'une quelconque des revendications 1 ou 4, ledit moyen de retenue (11, 53) étant doté d'au moins une ouverture d'inspection (55) à travers laquelle la position de l'extrémité proximale du corps de stent (8) relativement au sphincter (5) peut être précisément déterminée par un endoscope (57) introduit dans un passage dans la tige de cathéter (20, 51, 52 ; 61) durant l'utilisation dudit cathéter (12, 50).

6. Cathéter (12, 50) selon la revendication 5, ladite ouverture d'inspection (55) s'étendant quelque peu à l'intérieur de l'extrémité proximale de la partie de la tige de cathéter (20, 51, 52 ; 61) entourée par le corps de stent (8) lorsqu'un stent (1') est disposé sur ledit cathéter (12, 50).

7. Système comprenant un stent (1') et un cathéter (12, 50), ledit stent (1') comprenant une première partie (8) à positionner dans une partie malade (2) de l'organe et y agissant comme un corps de stent, et une seconde partie (10, 10', 40) à positionner dans une partie non malade (6) de l'organe et y agissant comme un élément d'ancrage, lesdites deux parties (8 ; 10, 10', 40) étant conçues sous la forme d'éléments tubulaires interconnectés au niveau des extrémités se faisant face par une partie de connexion flexible, axialement rigide (9), au moins ladite seconde partie (10, 10', 40) ayant en mémoire une capacité à se dilater radialement après une augmentation de température, permettant ainsi l'ancrage de la première partie (8) à une distance déterminée de la première partie corporelle (2) de l'organe, ledit cathéter (12, 50) comprenant une tige de cathéter (20, 61, 51) avec une poignée (21) et un moyen de déploiement de stent au niveau de son extrémité proximale et une partie de retenue de stent (70) au niveau de son extrémité distale, ladite partie de retenue de stent (70) étant divisée en une partie de retenue de corps de stent distale, une partie de retenue d'élément d'ancrage proximale et entre celles-ci un moyen de retenue (11, 53) destiné à retenir de manière libérable ladite partie de connexion (9) du stent (1') lorsque ledit stent (1') est disposé sur ledit cathéter (12, 50), ledit moyen de retenue (11, 53) étant constitué par un manchon entourant ladite tige de cathéter (20, 51, 52 ; 61) et doté d'une fente s'étendant axialement (26) sur sa longueur, ladite fente (26) accueillant ladite partie de connexion (9) lorsqu'un stent (1) est disposé sur ledit cathéter (12, 50).

8. Système selon la revendication 7, ledit cathéter (12, 50) étant un cathéter selon l'une quelconque des revendications 1 à 6.
